# EUROPÄISCHE PATENTSCHRIFT

(11) **EP 0 563 749 B1**
(45) Veröffentlichungstag und Bekanntmachung des Hinweises auf die Patenterteilung: **03.01.1996**
(21) Anmeldenummer: 93104735.1
(22) Anmeldetag: 23.03.1993
(51) Int. Cl.: A61K 6/083

(54) **Automatisch anmischbares Mittel zur Anfertigung von Kronen und Brücken**
Automatically mixable product for making crowns and bridges
Composition à mélange automatique pour la fabrication de couronnes et bridges

(30) Priorität: 03.04.1992 DE 4211040
(43) Veröffentlichungstag der Anmeldung: 06.10.1993
(73) Patentinhaber: ERNST MÜHLBAUER KG, D-22547 Hamburg (DE)
(72) Erfinder: May, Ulrich, W-2083 Halstenbek (DE); Engelbrecht, Jürgen Dr., W-2000 Hamburg 76 (DE); Mühlbauer, Ernst, W-2000 Hamburg 52 (DE); Lein, Edgar, W-2000 Hamburg 60 (DE)
(74) Vertreter: Beil, Hans Chr., Dr.

(56) Entgegenhaltungen:
- EP-A- 0 032 249
- EP-A- 0 049 559
- EP-A- 0 438 629
- WO-A-81/01366

## Beschreibung

Die Erfindung betrifft ein provisorisches Kronen- und Brückenmaterial, welches aus zwei Pasten besteht, die in einem statischen Mischapplikationsgerät in einem Gang angemischt und leicht ausgepreßt werden können.

Provisorische Kronen- und Brückenmaterialien werden verwendet, um für den Patienten eine Krone oder Brücke anzufertigen, die nur während eines kurzfristigen Zeitraums getragen wird.

Klassisch bestehen diese Materialien aus einer Pulver/Flüssigkeit-Kombination, welche zusammengemischt wird. Die Flüssigkeit besteht dabei im wesentlichen aus einem monofunktionellen Monomer (z.B. Methylmethacrylat) und Aktivator, das Pulver aus Polymer (z.B. Polymethylmethacrylat) und Katalysator.

Systeme dieser Art sind leicht zu handhaben, und die daraus hergestellten Kronen und Brücken sind ausgezeichnet bruchfest.
Die Flüssigkeiten sind jedoch nicht unproblematisch. Sie verdampfen leicht und besitzen einen unangenehmen Geruch. Zudem zeigen diese Systeme beim Polymerisieren eine potentiell pulpenschädigende Wärmeentwicklung und ein starkes Schrumpfverhalten.

Fortentwickelte Materialien bestehen deshalb aus zwei Pasten, die jeweils difunktionelle Methacrylate und Füllstoffe sowie, auf die Pasten getrennt aufgeteilt, Aktivator und Katalysator enthalten.
Sie können einfach zusammengemischt werden, riechen kaum, zeigen ein deutlich günstigeres Schrumpfverhalten und eine geringere Wärmeentwicklung bei der Aushärtung.
Ein sehr großes Problem jedoch ist, daß beim Anmischen auf dem Mischblock zahlreiche Luftblasen inkorporiert werden, welche später zu Rauhigkeiten und zu starken Verfärbungen im ausgehärteten Material führen. Zudem sind diese Materialien wegen ihrer Härte sehr spröde und neigen leicht zu Bruchverhalten.

Aufgabe der Erfindung war es deshalb, ein provisorisches Kronen- und Brückenmaterial zu finden, welches aus zwei Pasten besteht, ohne Einschluß von Blasen anmischbar ist und die genannte Sprödigkeit nicht aufweist.

Überraschend zeigte sich nun, daß provisorische Kronen- und Brückenmaterialien, die diesen Anforderungen entsprechen, erhalten werden können, wenn sie dadurch gekennzeichnet sind, daß
a) die eine Paste aus difunktionellen Acrylaten, Aktivatoren und röntgenopakem Füllstoff besteht,
b) die zweite Paste aus nicht polymerisierbarem (inertem) Weichmacher, Katalysator und Strukturbildner besteht,
c) das Mischungsverhältnis der Pasten gemäß a) und gemäß b) zwischen 20:1 und 5:1 liegt und daß
d) die Acrylate der Paste a) eine Viskosität unter 50 Pa.s (500 P) aufweisen und die Viskosität der Weichmacher der Paste b) unter der der Acrylate der Paste a) liegt, wobei beide Pasten ein solches rheologisches Verhalten aufweisen, daß sie in einem statischen Mischapplikationsgerät in einem Gang angemischt und leicht ausgepreßt werden können.

Solche Materialien, solchermaßen angemischt, können direkt in einen angefertigten Situationsabdruck zwecks Applikation am Zahnstumpf im Munde eingebracht werden. Dort polymerisieren sie dann unter Aushärtung und können anschließend bearbeitet werden.

Die solchermaßen hergestellten provisorischen Kronen und Brücken sind blasenfrei hergestellt, von hoher Ästhetik und ausgezeichnet bruchfest. Die Polymerisationsschrumpfung erweist sich als extrem niedrig. Die Röntgenopazität entspricht den klinischen Anforderungen.

Geeignete Pasten gemäß a) enthalten difunktionelle Acrylate, wie z.B. Bisphenol-A-glycidyldiacrylat, Bisphenol-A-diglycidyl-di-acyrylat, Triethylenglycoldiacrylat, Hexandioldiacrylat und Urethandiacrylate.

Als Aktivatoren sind tertiäre Amine, wie z.B. Triethanolamin, Dimethylaminobenzosäureester, Dihydroxyethyltoluidin und Kupfernaphthenat geeignet.
Als besonders geeignet haben sich als difunktionelles Acrylat Bisphenol-A-diglycidyldiacrylat und als Aktivator Dimethyl-p-toluidin erwiesen.

Als röntgenopake Füllstoffe eignen sich Pulver unlöslicher Gläser, die ausreichende Mengen an Schwermetallen, wie Yttrium, Zirkonium oder Lanthan, enthalten. Ästhetisch besonders befriedigende Ergebnisse bei guter Röntgenopazität werden mit Barium- und/oder Strontiumgläsern erhalten.

Geeignete Pasten gemäß b) enthalten Weichmacher, die, um eine hohe Flexibilität zu erhalten, nicht mit einpolymerisieren können, aber andererseits ausreichend wenig im Milieu des Mundes löslich sind. Geeignet sind flüssige Paraffine, langkettige Glycole und inerte Alkylphthalate. Besonders gut geeignet sind mittelmolekulare Polyethylenglycole wie Polyethylenglycol-200 oder Polyethylenglycol-400.

Geeignete Katalysatoren sind Peroxide, wie z.B. Dibenzoylperoxid, Methylethylketonperoxid, Cumolhydroperoxid wie auch Malonylsulfamide, wie sie in der EP 0 059 451 beschrieben sind, oder autoxidativ wirkende CH-aktive Verbindungen, wie sie in "Makromolekulare Chemie" 99 (1966), 96-102 beschrieben sind.
Besonders geeignet ist die Verwendung von Bis-dichlorbenzoylperoxid als Katalysator.

Die Systeme können zusätzlich auch lichthärtbar sein, wie sie z.B. in US-P 4,071,424 oder in DE 31 36 484 beschrieben sind.

Als Strukturbildner sind amorphe Siliciumdioxidmodifikationen vorteilhaft. Beispiele sind pyrogene und gefällte Kieselsäuren sowie Kieselgur.
Besonders geeignet sind agglomerierte pyrogene Kieselsäuren oder gesintertes Kieselgel, wie sie in den Patenten EP 0 040 232 und EP 0 113 926 beschrieben sind.
Solche Füllstoffe neigen wenig zum Nachdicken und haben die Eigenschaft, daß ihr thixotropes Verhalten gleichmäßig ist und kaum von der Dauer der Lagerung der Pasten abhängt. Dadurch kann ein dauerhaftes, gleichbleibendes Mischergebnis gewährleistet sein. Auch kann dadurch der Auspreßdruck, der sehr kontrolliert sein muß, konstant gehalten werden.

Das Mengenverhältnis bei den zu vermischenden Pasten kann in einem Rahmen von 20:1 bis 5:1 (Paste gemäß a) zu Paste gemäß b)) variieren. In den genannten Bereichen führt der zugegebene Weichmacher zu besonders günstigen bruchfesten Materialien.
Außerhalb dieser Grenzen werden die Materialien zu wenig flexibel und spröde oder viel zu weich und unbeständig im Mundmilieu.

Es ist schließlich auch auf das rheologische Verhalten der beiden Pasten zu achten. Bekannt ist das Anmischen in statischen Mischern von zwei Flüssigkeiten oder Pasten mit gleichen oder ähnlichen Konsistenzen und in gleichen Mengenverhältnissen. Es liegt aber in der Natur der erfindungsgemäßen Materialien, daß sie ein anderes Mischungsverhältnis erfordern. Es wurde aber gefunden, daß ein Mischungsverhältnis von etwa 10:1 nur dann zu einem befriedigenden Mischungsergebnis führt, wenn die Konsistenz der im geringeren Verhältnis zugesetzten Paste dünner eingestellt wird. Befriedigende Durchmischung von Pasten gleicher oder ähnlicher Konsistenz kann durch eine spezielle Gestaltung des statischen Mischers allein nicht erreicht werden, da die Pasten eine vom Anwendungszweck her gebotene Mindeststandfestigkeit haben müssen, aus ökonomischen Gründen die Größe des Mischers begrenzt ist und aus anwendungstechnischen Gründen die Mischzeit kurz sein muß.

Um die angestrebte Konsistenz zu erreichen, werden die Acrylate der einen Paste a) so gewählt, daß ihre Viskosität unter 50 Pa s (500 **P**) liegt. Die Weichmacher der anderen Paste b) müssen so gewählt werden, daß ihre Viskosität unter der der oben genannten Acrylate liegt. Um die vom Anwendungszweck her erforderliche Mindeststandfestigkeit zu erreichen ohne die Mischergängigkeit zu verschlechtern, sollten auch Mittel zur Erhöhung der Standfestigkeit, wie z.B. pyrogene Kieselsäure, zugesetzt werden.

Besonders leicht ist die Handhabbarkeit. Nach Aufsetzen einer Mischkanüle auf ein Mischapplikationsgerät, wie sie in EP 0 232 733 und EP 0 261 466 beschrieben sind, muß nur noch ein Vorschub bedient werden. Das Material kommt genau im richtigen Mischverhältnis und homogen angemischt aus der statischen Mischkanüle heraus. Die vorher blasenfreien Pasten sind auch noch nach dem Mischen blasenfrei und ergeben somit auch blasenfreie Polymerisate. Die so hergestellten Kronen und Brücken sind von hoher Ästhetik und ausgezeichnet bruchfest.
Ihre Polymerisationsschrumpfung erweist sich als extrem niedrig. Ihre Röntgenopazität entspricht den klinischen Anforderungen.

Das nachstehend aufgeführte Beispiel soll ein erfindungsgemäßes Material beschreiben.

### Beispiel

Es werden folgende Komponenten zu einer Katalsysator-Paste gerührt:

| | |
|---|---|
| Polyethylenglycol-400 | 48,0 Gew.-Teile |
| Dibutylphthalat | 12,0 Gew.-Teile |
| Dichlordibenzoylperoxid | 8,0 Gew.-Teile |
| Gesintertes Kieselgel | 32,0 Gew.-Teile. |

Entsprechend werden folgende Komponenten zu einer Basis-Paste gerührt:

| | |
|---|---|
| Bisphenol-A-diglycidyldiacrylat | 50,0 Gew.-Teile |
| Tripropylenglycoldiacrylat | 7,0 Gew.-Teile |
| Strontiumglas, silanisiert | 39,0 Gew.-Teile |
| Silanisierte pyrogene Kieselsäure | 3,0 Gew.-Teile |
| Dimethyl-p-toluidin | 1,0 Gew.-Teile |
| Hydrochinonmonomethylether | 0,01 Gew.-Teile |
| Eisenoxid-Pigment | 0,02 Gew.-Teile |

Basispaste und Katalysatorpaste werden getrennt in die Kammern einer Doppelkartusche entsprechend EP 0 261 466 gefüllt, die Doppelkartusche mit einer Mischkanüle versehen und unter Verwendung einer Austragsvorrichtung entsprechend EP 0 232 733 durch die Mischkanüle in einen vorgefertigten Situationsabdruck direkt appliziert. Darauf wird der Situationsabdruck inklusive gemischten Materials sofort auf einen präparierten Zahnstumpf aufgebracht. Nach 3 Minuten kann der Situationsabdruck entfernt werden. Die auf dem Zahnstumpf verbleibende ausgehärtete Krone wird abgehebelt und außerhalb des Mundes ausgearbeitet.
Nach einer Politur wird eine ästhetisch sehr ansprechende blasenfreie Krone von hoher Bruchfestigkeit erhalten.

## Patentansprüche

1. Aus zwei Phasen bestehendes röntgenopakes Material zur Anfertigung von provisorischen Kronen und Brücken, dadurch gekennzeichnet, daß
a) die eine Paste aus difunktionellen Acrylaten, Aktivatoren und röntgenopakem Füllstoff besteht,
b) die zweite Paste aus nicht polymerisierbarem (inertem) Weichmacher, Katalysator und Strukturbildner besteht,
c) das Mischungsverhältnis der Pasten gemäß a) und gemäß b) zwischen 20:1 und 5:1 liegt und daß
d) die Acrylate der Paste a) eine Viskosität unter 50 Pa s (500 P) aufweisen und die Viskosität der Weichmacher der Paste b) unter der der Acrylate der Paste a) liegt, wobei beide Pasten ein solches rheologisches Verhalten aufweisen, daß sie in einem statischen Mischapplikationsgerät in einem Gang angemischt und leicht ausgepreßt werden können.

2. Material nach Anspruch 1, dadurch gekennzeichnet, daß das difunktionelle Acrylat Bisphenol-A-diglycidyldiacrylat ist.

3. Material nach Ansprüchen 1 bis 2, dadurch gekennzeichnet, daß der Aktivator Dimethyl-p-toluidin ist.

4. Material nach Ansprüchen 1 bis 3, dadurch gekennzeichnet, daß der röntgenopake Füllstoff Barium- und/oder Strontiumglas ist.

5. Material nach Ansprüchen 1 bis 4, dadurch gekennzeichnet, daß der nicht polymerisierbare Weichmacher Polyethylenglycol-200 oder Polyethylenglycol-400 ist.

6. Material nach Ansprüchen 1 bis 5, dadurch gekennzeichnet, daß der Katalysator Bisdichlorbenzoylperoxid ist.

7. Material nach Ansprüchen 1 bis 6, dadurch gekennzeichnet, daß es zusätzlich lichthärtbar ist.

8. Material nach Ansprüchen 1 bis 7, dadurch gekennzeichnet, daß der Strukturbildner agglomerierte pyrogene Kieselsäure oder gesintertes Kieselgel ist.

9. Material nach Ansprüchen 1 bis 8, dadurch gekennzeichnet, daß das Mischungsverhältnis der Pasten gemäß a) und gemäß b) 10:1 ist.

10. Mischverfahren, dadurch gekennzeichnet, daß ein Material nach den Ansprüchen 1 bis 9 in einem statischen Mischapplikationsgerät angemischt und direkt aus diesem appliziert wird.

## Claims

1. Material which is opaque to X-rays consisting of two pastes for the production of provisional crowns and bridges, characterised in that
a) one paste consists of difunctional acrylates, activators and filler that is opaque to X-rays,
b) the second paste consists of non-polymerisable (inert) plasticiser, catalyst and structure-forming agent,
c) the mixing ratio of the pastes according to a) and according to b) lies between 20:1 and 5:1 and that
d) the acrylates of paste a) have a viscosity below 50 Pa.s (500 P) and the viscosity of the plasticisers of paste b) lies below that of the acrylates of paste a), both pastes exhibiting such rheological behaviour that they can be mixed in a static mixer/applicator in one operation and easily extruded.

2. Material according to Claim 1, characterised in that the difunctional acrylate is Bisphenol-A diglycidyl diacrylate.

3. Material according to Claims 1 to 2, characterised in that the activator is dimethyl-p-toluidine.

4. Material according to Claims 1 to 3, characterised in that the filler which is opaque to X-rays is barium glass and/or strontium glass.

5. Material according to Claims 1 to 4, characterised in that the non-polymerisable plasticiser is polyethyleneglycol-200 or polyethyleneglycol-400.

6. Material according to Claims 1 to 5, characterised in that the catalyst is bis-dichlorobenzoyl peroxide.

7. Material according to Claims 1 to 6, characterised in that it is additionally capable of being cured under the influence of light.

8. Material according to Claims 1 to 7, characterised in that the structure-forming agent is agglomerated pyrogenic silicic acid or sintered silica gel.

9. Material according to Claims 1 to 8, characterised in that the mixing ratio of the pastes according to a) and according to b) is 10:1.

10. Mixing process, characterised in that a material according to Claims 1 to 9 is mixed in a static mixer/applicator and applied directly from the latter.

## Revendications

1. Matière opaque aux rayons X se composant de deux phases, destinée à la fabrication de couronnes et de bridges provisoires, caractérisée en ce que
a) la première pâte se compose d'acrylates bifonctionnels, d'activateurs et d'une masse de remplissage opaque aux rayons X,
b) la deuxième pâte se compose d'un plastifiant non polymérisable (inerte), d'un catalyseur et d'un agent générateur de structure,
c) le rapport des ingrédients des pâtes selon a) et selon b) se situe entre 20 : 1 et 5 : 1 et en ce que
d) les acrylates de la pâte a) présentent une viscosité inférieure à 50 Pas (500 P) et que la viscosité des plastifiants de la pâte b) se situe au-dessous de celle des acrylates de la pâte a), les deux pâtes présentant un comportement rhéologique tel qu'elles peuvent être mélangées en une fois dans un appareil d'application de mélange statique et facilement comprimées.

2. Matière selon la revendication 1, caractérisée en ce que l'acrylate bifonctionnel est un bisphenol-A diglycidyldiacrylate.

3. Matière selon les revendications 1 à 2, caractérisée en ce que l'activateur est du diméthyl-p-toluidine.

4. Matière selon les revendications 1 à 3, caractérisée en ce que la masse de remplissage opaque aux rayons X est du verre au baryum et/ou strontium.

5. Matière selon l'une des revendications 1 à 4, caractérisée en ce que le plastifiant non polymérisable est du polyéthylèneglycol-200 ou du polyéthylèneglycol-400.

6. Matière selon les revendications 1 à 5, caractérisée en ce que le catalyseur est du peroxyde de bis-dichlorobenzène.

7. Matière selon les revendications 1 à 6, caractérisée en ce qu'elle est de plus durcissable à la lumière.

8. Matière selon les revendications 1 à 7, caractérisée en ce que l'agent générateur de structure est un gel de silice pyrogéné aggloméré ou un gel de silice fritté.

9. Matière selon les revendications 1 à 8, caractérisée en ce que le rapport des ingrédients des pâtes selon a) et selon b) est de 10 : 1.

10. Procédé de mélange, caractérisé en ce que une matière est mélangée selon les revendications 1 à 9 dans un appareil d'application de mélange statique et est directement appliquée à la sortie de celui-ci.
